(19) **Europäisches Patentamt**

**European Patent Office**

**Office européen des brevets**

(11) Veröffentlichungsnummer: **0 164 483**
**B1**

(12) # EUROPÄISCHE PATENTSCHRIFT

(45) Veröffentlichungstag der Patentschrift:
**16.08.89**

(51) Int. Cl.⁴: **A 61 L 27/00** // A61F2/28,
A61L25/00

(21) Anmeldenummer: **84810288.5**

(22) Anmeldetag: **12.06.84**

(54) **Verfahren zur Herstellung von Knochenersatzmaterial.**

(43) Veröffentlichungstag der Anmeldung:
**18.12.85 Patentblatt 85/51**

(45) Bekanntmachung des Hinweises auf die Patenterteilung:
**16.08.89 Patentblatt 89/33**

(84) Benannte Vertragsstaaten:
**CH DE FR GB IT LI**

(56) Entgegenhaltungen:
**EP-A- 0 016 906**
**EP-A- 0 030 583**
**EP-A- 0 048 558**
**EP-A- 0 082 621**
**US-A- 3 443 261**

**CHEMICAL ABSTRACTS, Band 96, Nr. 20, 17. Mai 1982,
Seite 407, Nr. 168778r, Columbus, Ohio, US; & RO - A -
69 198 (INSTITUTUL DE CERCETARI PIELARIE SI
INCALTAMINTE) 08.10.1980**

(73) Patentinhaber: **OSCOBAL AG, Bohnackerweg 1,
CH-2545 Selzach (CH)**

(72) Erfinder: **Ries, Peter, Dr., Unterer Rebbergweg 123,
CH-4153 Reinach (CH)**
Erfinder: **Mittelmeier, Heinz, Prof. Dr., Am Gedünner,
D-6650 Homburg-Schwarzenbach (DE)**
Erfinder: **Baumgart, Frank, Prof. Dr. Ing., Rebgasse 92,
CH-2540 Grenchen (CH)**

(74) Vertreter: **Seehof, Michel et al, c/o AMMANN
PATENTANWAELTE AG BERN Schwarztorstrasse 31,
CH-3001 Bern (CH)**

**Beschreibung**

Die vorliegende Erfindung bezieht sich auf ein Verfahren zur Herstellung von Knochenersatzmaterial, aus Kollagen enthaltendem Material und einer mineralischen Komponente, wobei das Kollagen enthaltende Material vernetzt wird. Ein solches Verfahren ist summarisch im Chemical Abstracts, Band 96, Nr. 20, 17. Mai 1982, Seite 407, Nr. 168778 r, beschrieben. Das darin beschriebene Material wird aus zwei verschiedenartigen Kollagenen, Kollagenhydrolysat, d. h. Gelatine, und Calciumphosphat mittels viel Gerbmittel hergestellt. Kollagenderivate und speziell fibrilläre Kollagene, die nicht über eine enzymatische Auflösung der Kollagenfasern gereinigt wurden, weisen bekanntlich eine antigene Potenz auf, die im Organismus zu Unverträglichkeiten führt. Calciumphosphat in ungesinterter Form weist Beimengungen von stark alkalisch reagierenden Verunreinigungen auf, die ebenfalls nach der Implantation zu unerwünschten Nebenwirkungen und Abwehrreaktionen Anlaß geben. Außerdem sollen diese Strukturen als Prothesen dienen und weisen ganz andere Eigenschaften auf als das angestrebte Knochenersatzmaterial. Außerdem müssen vorgängig drei verschiedene Kollagenderivate hergestellt werden. Schließlich wird in dieser Entgegenhaltung das Material mit 1,5% Formaldehyd gegerbt. Dadurch wird das Material so stark denaturiert, daß es kunststoffartig wird und nach der Implantation vom Organismus nicht resorbiert, sondern als Fremdkörper erkannt wird, was bekanntlich unerwünschte Nebenwirkungen hervorruft. Außerdem wird eine Stimulation des Knochenwachstums für das Material nicht beschrieben.

Ferner ist das eingangs erwähnte Verfahren in der EP-A0 030 583 des gleichen Anmelders beschrieben. Ausgedehnte klinische Untersuchtungen wurden in der Zeitschrift für Orthopädie 121 (1983), Seite 115–123, mit dem Titel «Klinische Erfahrungen mit Kollagen-Apatit-Implantation zur lokalen Knochenregeneration», H. Mittelmeier, B.D. Katthagen, veröffentlicht, woraus hervorgeht, daß ermutigende und zufriedenstellende Erfolge damit erzielt werden konnten. Diese Untersuchungen ergaben ferner, daß das Material trotz Wärmebehandlung und HCL-Bedampfung klinisch beim Einbringen ins Gewebe, insbesondere Tränkung mit Blut und Gewebeflüssigkeit, noch relativ zerfließlich und die Knochenbildung noch nicht massiv genug ist. Deshalb wäre ein festeres Material wünschenswert, welches zudem noch eine bessere Stimulation des Knochenwachstums hervorruft.

In der EP-A036 415 ist ein Verfahren zur Herstellung eines Kollagenproduktes beschrieben, das nach Zumischung von feinem Apatitpulver als Knochenersatzmaterial für die oben genannten Versuche diente. Damit konnte eine wesentliche Steigerung der Saugfähigkeit und der mechanischen Festigkeit über vorbekannte Kollagenvliese dadurch erreicht werden, daß das Kollagenprodukt einer zusätzlichen Wärmebehandlung oder einer Behandlung mit gasförmigem Halogenwasserstoff unterworfen wurde.

Aus der gleichen Veröffentlichung Seite 10, Zeile 23, bis Seite 11, Zeile 3, geht ferner hervor, daß es an sich bekannt war, die physikalisch-chemischen Eigenschaften von Kollagen oder Gelatineprodukten auf chemischem Wege, z.B. durch Vernetzen mit Aldehyden, insbesondere Formaldehyd oder Glutaraldehyd, zu verbessern. Diese Behandlungsmethoden hätten jedoch den Nachteil, daß die erhaltenen Produkte bei der medizinischen Implantation vom Körper nur sehr langsam oder überhaupt nicht resorbiert werden und vor allem im Körper Entzündungen, Abwehrreaktionen oder die Bildung von Fremdkörperriesenzellen verursachen. Das Vernetzen der Kollagenprodukte mit Aldehyden wurde dabei erschöpfend durchgeführt, so daß vollständig vernetzte, kunststoffartige Produkte resultierten, die vom Organismus als Fremdkörper erkannt werden und so die erwähnten schädlichen Reaktionen hervorrufen. Restmengen von ungebundenem Aldehyd im Implantat sind ebenfalls gewebeschädigend.

Aus der Veröffentlichung Jap. Traumat. Surg. (1982) 99, S. 265–269 von K. Hayashi et al. ist ein Kollagen-Hydroxylapatit-Präparat bekannt, das nach Quervernetzung gefriergetrocknet wurde. Einerseits wurde relativ feinkörniges Apatit verwendet und andererseits keinerlei Hinweis darauf gegeben, daß die Beimengung von Glutaraldehyd derart sein sollte, daß keine der oben beschriebenen Reaktionen eintreten.

Es ist demgegenüber Aufgabe der vorliegenden Erfindung ein Verfahren zur Herstellung von Knochenersatzmaterial anzugeben, mit welchem es möglich ist, ein festeres, wenig zerreißliches Knochenersatzmaterial herzustellen, das das An- und Hineinwachsen des Knochens noch mehr begünstigt und keinerlei unerwünschte Nebenwirkungen hervorruft. Die Aufgabe wird mit einem in den Ansprüchen beschriebenen Verfahren gelöst.

Es wurde nun überraschend gefunden, daß durch eine nur partielle Vernetzung des Kollagens mit Formaldehyd oder Glutaraldehyd eine ganz erhebliche Verbesserung der physikalisch-chemischen Eigenschaften des Kollagens hervorgerufen werden konnte, ohne daß andererseits das fertige Produkt nach Implantation im Organismus negative Fremdkörperreaktionen auslöst, wie sie zuvor beschrieben wurden, oder Schäden durch Restmengen von Aldehyd auftreten.

Bei der Herstellung des Knochenersatzmaterials, das den obgenannten Versuchen zugrunde lag, wurde ein sehr feines Apatitpulver bzw. Tricalciumphosphat mit Partikeln in der Größe von 3–5 μm verwendet. Es wurde nun gefunden, daß die osteogenetische Wirkung des Apatits wesentlich erhöht wird, falls dieses in Form von Pulveraggregaten oder größeren Teilchen vorliegt. Diese Teilchenvergrößerung kann vorzugsweise dadurch erreicht werden, daß handelsübliches, in feinkristalliner Form vorliegendes Apatit bzw. Tricalciumphosphat zu Körnern von etwa 20–1000 μm, vorzugsweise von 50–300 μm Durchmesser, gesintert werden.

Inzwischen durchgeführte Versuche haben ergeben, daß ein Knochenersatzmaterial, das aus partiell mit Formaldehyd vernetztem Kollagen und Apatit bzw. Tricalciumphosphat in Form gesinterter Körner, die bereits der Kollagenlösung vor der Lyophilisation beigefügt werden, besteht, erheblich bessere Resultate erbringt als das vorbekannte Knochenersatzmaterial, wodurch ein solches neues Knochenersatzmaterial für ein größeres Indikationsgebiet geeignet ist. Als besonders vorteilhaft erweist sich, die Verteilung der Apatitkörner im Kollagen analog der Verteilung der Kreuzungspunkte der Mineralstrukturen im natürlichen Knochen zu wählen, so daß bei Beginn der Neubildung von Knochen das Knochenersatzmaterial als eine Art Matrix vorliegt, die der des zu bildenden Knochens ähnlich ist.

Das zur Durchführung des erfindungsgemäßen Verfahrens benötigte Ausgangsmaterial kann in der nachstehend beschriebenen Weise hergestellt werden:

1 kg Rindersehnen wurde bei − 10 bis − 20°C eingefroren und während 10–20 Min. mit einem Messerhomogenisator hoher Tourenzahl sehr fein zerkleinert. Die Temperatur des Mahlgutes wurde durch Zugabe von Eisstückchen unter + 40°C gehalten. Der erhaltene zähe fasrige Gewebebrei wurde in 5 Liter 10%iger NaCl-Lösung, die 2,5 g Natriumazid und 50 ml einer 10%igen wäßrigen Lösung des nichtionischen Netzmittels NP 55/52 (Polyoxyäthylennonoylphenyläther) enthielt, unter kräftigem Rühren suspendiert. Die Suspension wurde 2 h bei Raumtemperatur weitergerührt und anschließend zentrifugiert. Der grau bis bräunlich gefärbte trübe Überstand, der Fett und unerwünschte wasserlösliche Ballaststoffe enthielt, wurde verworfen. Der zurückbleibende weiße Hautfaserbrei wurde noch zweimal in der gleichen Weise extrahiert, wobei der Extraktionsflüssigkeit pro Liter 0.1 Mol Dinatriumhydrogenphosphat zugesetzt wurde.

Als Ausgangsmaterial kann man anstelle von Rindersehnen auch Schweinehaut verwenden.

Ein in der oben beschriebenen Weise aus 1 kg Rindersehnen erhaltener entfetteter und extrahierter fasriger Gewebebrei wurde im 5fachen Volumen 0,5 M Essigsäure suspendiert. Der Suspension wurde eine Lösung von 1 g technischem Pepsin in 100 ml 0.01 N HCl zugesetzt. Der pH der Suspension wurde mit HCl auf 2.9 eingestellt. Die Suspension wurde unter wiederholtem Rühren während 48 h digeriert. Die zähflüssige Kollagenlösung wurde durch einen Saugfilter G 1 filtriert, um undigerierte Rückstände zu entfernen. Das Kollagen wurde durch Zugabe von 30%iger wässriger Natriumhydroxydlösung aus der Suspension ausgefällt und durch Zentrifugieren abgetrennt. Das Kollagen wurde durch Lösen in 0,5 M Essigsäure und Ausfällen durch langsames Zugeben von 3% Natriumchlorid gereinigt. Das gereinigte Kollagen wurde in 0,5 M Essigsäure gelöst und mit Wasser verdünnt. Das im Kollagen noch vorhandene restliche Natriumchlorid wurde durch Waschen auf einem Ultrafilter entfernt. Die Ultrafiltration wurde so lange fortgesetzt, bis im Eluat bei Zugabe von Silbernitrat keine Chloridionen mehr nachweisbar waren und die Kollagenkonzentration etwa 1% betrug. Die Kollagenlösung wurde filtriert. mit 0.2 Gew.-% Formaldehyd und 5 Gewichtsteilen gesinterten Apatitkörnern der Größe 20–1000 µm. beides bezogen auf den Kollagengehalt der Kollagenlösung. versetzt. ca. 20 Minuten gerührt. in geeignete Formen gegossen. mehrere Stunden stehen gelassen. lyophilisiert und danach durch γ-Bestrahlung sterilisiert.

Beispiel 1

600 g einer nach der oben beschriebenen Methode hergestellten, ultrafiltrierten Kollagenlösung mit einem Kollagengehalt von 0,88 Gew.-% wurde klarfiltriert und mit 10,6 ml einer 0,1 Gew.-%igen wässrigen Formaldehydlösung versetzt und gerührt. Unter Rühren wurden der Mischung 26,4 g gesinterte Apatitkörner mit ca. 50–150 µm Durchmesser zugegeben und während 22 Minuten gerührt. Der pH der Lösung stieg während des Rührens von ursprünglich pH 3,5 auf 6,35 an. Die erhaltene Mischung wurde in Portionen zu 70 g in Polystyrolschalen der Größe 12 × 7,5 cm gegossen, über Nacht stehen gelassen. am anderen Tag lyophilisiert, danach verpackt und schließlich durch γ-Bestrahlung mit einer Dosis von 2,5 Mrad sterilisiert.

Beispiel 2

800 g einer nach der oben beschriebenen Methode hergestellten, ultrafiltrierten Kollagenlösung mit einem Kollagengehalt von 1.07 Gew.-% Kollagen wurde mit 6,83 ml einer 0,25 Gew.-%igen, wässrigen Glutaraldehydlösung und mit 17,12 g = der doppelten Menge. bezogen auf den Kollagengehalt, an gesinterten Apatitkörnern bzw. Tricalciumphosphat mit ca. 300–700 µm Durchmesser versetzt, eine halbe Stunde gerührt, 10 Stunden stehen gelassen, in Portionen zu 18 g in Schalen der Größe 6.4 × 3.8 cm gefüllt, lyophilisiert, verpackt und durch Bestrahlung mit 2,5 Mrad γ-Strahlen sterilisiert.

Beispiel 3

200 g einer nach der oben beschriebenen Methode hergestellten, ultrafiltrierten Kollagenlösung mit einem Kollagengehalt von 0,97 Gew.-% wurden mit 5,82 g = der dreifachen Menge. bezogen auf den Kollagengehalt. an gesinterten Apatitkörnern von ca. 100 µm Durchmesser gemischt, eine halbe Stunde gerührt, in Portionen zu 40 g in runde Schalen mit einem Durchmesser von 8 cm abgefüllt und lyophilisiert. Das erhaltene Material war gegenüber wäßrigen Lösungen unbeständig und wurde daher partiell vernetzt, indem es in einer, mit 35 Gew.-%iger Formaldehydlösung beschickten. geschlossenen Kammer der Formaldehydgasphase während 2 Stunden ausgesetzt wurde. Danach wurde die Formaldehydlösung aus der Kammer entfernt und die Kammer 6mal evakuiert und belüftet. um das nicht gebundene Formaldehydgas aus dem Kollagen-Apatit-Material zu entfernen. Bei der dritten Belüftung wurde die Luft durch eine wäßrige Ammoniumhydroxydlösung durchgeleitet, um letzte Restmengen des Formaldehyds an den einströmenden Ammoniak zu binden. Das erhaltene Knochenersatzmaterial war nach dieser Behandlung in wäßrigen Lösungen beständig und nach der Sterilisation für die Implantation in Knochendefekte geeignet.

Aus obigem Beispiel geht hervor, daß man gute Ergebnisse mit einer Menge Formaldehyd erreichen kann. die unter 1 Gew.-%. bezogen auf das Trockengewicht des Kollagens liegt. vorzugsweise in einer Menge von 0.05–0.5 Gew.-%. Bei der Verwendung anderer Aldehyde ist die entsprechende Menge zu verwenden.

Außer den genannten Form- und Glutaraldehyden können auch ein gesättigter oder ungesättigter, mono- oder polyfunktioneller aliphatischer Aldehyd, Glyoxal, unsubstituierte oder substituierte Di- oder Trichlortriazine verwendet werden, wobei als gasförmiges Vernetzungsmittel auch Äthylenoxyd in Frage kommt.

Es ist zweckmäßig, die mechanische Festigkeit des Knochenersatzmaterials noch weiter zu verstärken, indem man es mit wabenförmigen, netzartigen, schichtartigen oder textilgewebeähnlichen (gewobenen, gezwirnten, gestrickten) Stützelementen aus Kunststoff, Textil, Metall, Aluminiumoxyd, Keramiken, Kohlenstoffasergewebe, Kohlenstoff, Knochenzement, Glasfaser oder Bioglasfaser kombiniert. Die Stützelemente können die Form des zu ersetzenden Knochenteils aufweisen. Es kann auch zweckmäßig sein, dem neuen Knochenersatzmaterial Antibiotika beizugeben.

Zwecks Verdichtung und Verfestigung kann das Knochenersatzmaterial im trockenen oder nassen Zustand, gegebenenfalls unter Erwärmung komprimiert werden.

## Patentansprüche

1. Verfahren zur Herstellung von Knochenersatzmaterial aus Kollagen enthaltendem Material und einer mineralischen Komponente, wobei das Kollagen enthaltende Material vernetzt wird, dadurch gekennzeichnet, daß eine wäßrige Lösung oder Dispersion von gereinigtem Kollagen verwendet wird, die mit der aus Apatit und/oder Hydroxylapatit und/oder Calciumphosphat-Keramik bestehenden mineralischen Komponente und einer eine partielle Vernetzung des Kollagens bewirkenden Menge eines Vernetzungsmittels gemischt wird, wobei die Menge des verwendeten Vernetzungsmittels so bemessen wird, daß es der Menge von weniger als 1 Gew.-% Formaldehyd, bezogen auf das Trockengewicht des Kollagens, äquivalent ist, derart, daß das Material nach Implantation im Körper keine unerwünschten Nebenwirkungen hervorruft und es seine Resorbierbarkeit und sein Absorptionsvermögen gegenüber Körperflüssigkeiten beibehält; und die Mischung lyophilisiert wird.

2. Verfahren nach Anspruch 1, dadurch gekennzeichnet, daß als mineralische Komponente Apatit, insbesondere gesinterte Hydroxylapatite bzw. Calciumphosphate mit einem Durchmesser von 20–1000 μm, vorzugsweise 50–300 μm verwendet werden.

3. Verfahren nach Anspruch 1 oder 2, dadurch gekennzeichnet, daß das Gewichtsverhältnis von Kollagen zu mineralischer Komponente 1:2 bis 1:10, vorzugsweise 1:3 bis 1:5 beträgt.

4. Verfahren nach einem der Ansprüche 1–3, dadurch gekennzeichnet, daß man eine wäßrige Lösung oder Dispersion des gereinigten Kollagens mit den Körnern der mineralischen Komponente mischt, die Mischung lyophilisiert und das erhaltene Lyophilisat mit gasförmigem Vernetzungsmittel behandelt.

5. Verfahren nach Anspruch 4, dadurch gekennzeichnet, daß man als gasförmiges Vernetzungsmittel gasförmigen Formaldehyd oder Äthylenoxid verwendet.

6. Verfahren nach Anspruch 1, dadurch gekennzeichnet, daß man als Vernetzungsmittel einen gesättigten oder ungesättigten, mono- oder polyfunktionellen aliphatischen Aldehyd verwendet.

7. Verfahren nach Anspruch 6, dadurch gekennzeichnet, daß man als Aldehyd Formaldehyd, Glutaraldehyd oder Glyoxal verwendet.

8. Verfahren nach Anspruch 1, 6 oder 7, dadurch gekennzeichnet, daß man zur partiellen Vernetzung des Kollagens Formaldehyd in einer Menge von 0,05–0,5 Gew.-%, bezogen auf das Trockengewicht des Kollagens, oder die äquivalente Menge von einem der anderen genannten Aldehyde verwendet.

9. Verfahren nach Anspruch 1, dadurch gekennzeichnet, daß man als Vernetzungsmittel unsubstituierte oder substituierte Di- oder Trichlortriazine verwendet.

10. Verfahren nach einem der Ansprüche 1–9, dadurch gekennzeichnet, daß man dem Kollagen, der mineralischen Komponente oder dem daraus hergestellten Material ein Antibiotikum zusetzt.

11. Verfahren nach einem der Ansprüche 1–10, dadurch gekennzeichnet, daß man das Knochenersatzmaterial auf eine wabenförmige oder poröse Grundstruktur aus Kunststoff, Textil, Metall, Keramik, Kohlenstoffasergewebe oder Knochenzement aufschichtet.

12. Verfahren nach einem der Ansprüche 1–11, dadurch gekennzeichnet, daß man das Knochenersatzmaterial trocken oder naß, mit oder ohne Erwärmung mechanisch komprimiert.

## Claims

1. A method of producing a bone substitute material from a material containing collagen and a mineral component, the material containing the collagen being cross-linked, characterized in that one aqueous solution or dispersion of cleaned collagen is utilized which is mixed with the mineral component consisting of apatite and/or hydroxylapatite and/or calcium phosphate ceramics and a quantity of a cross-linking agent producing a partial cross linking of the collagen, the quantity of the cross-linking agent utilized being so dimensioned as to be equivalent to the quantity of less than 1% in weight of formaldehyde with respect to the dry weight of the collagen, so that the material, after implantation in the body, does not give rise to secundary, undesirable effects whilst retaining its resorbability and its absorbing capacity vis-a-vis bodily liquids; and in that the mixture is lyophilized.

2. A method according to claim 1, characterized in that apatite, more particularly sintered hydroxylapatite, resp. calcium phosphate with a diameter of 20–1000 μm, preferably 50–300 μm are utilized as mineral component.

3. A method according to claim 1 or 2, characterized in that the proportion in weight of the collagen to the mineral component is from 1:2 to 1:10, preferably from 1:3 to 1:5.

4. A method according to one of the claims 1 to 3, characterized in that one mixes an aqueous solution or dispersion of the cleaned collagen with the grains of the mineral component, that one lyophilises the mixture and in that one treats the obtained lyophilisate with a gazeous cross-linking agent.

5. A method according to claim 1, characterized in that one utilizes gazeous formaldehyde or ethylenoxide as gazeous cross-linking agent.

6. A method according to claim 1, characterized in that one utilizes as cross-linking agent a saturated or unsaturated, mono- or polyfunctional aliphatic aldehyde.

7. A method according to claim 6, characterized in that one utilizes as aldehyde formaldehyde, glutaraldehyde or glyoxal.

8. A method according to claim 1, 6 or 7, characterized in that for the partial cross-linking of the collagen, one utilizes formaldehyde in a quantity of 0.05–0.5% in weight with respect to the dry weight of the collagen or the equivalent quantity of one of the other aldehydes cited.

9. A method according to claim 1, characterized in that one utilizes as cross-linking agent an unsubstituted or substituted di- or trichlortriazine.

10. A method according to one of the claims 1–9, characterized in that one adds an antibiotic to the collagen, to the mineral component or to the material produced from them.

11. A method according to one of the claims 1–10, characterized in that one piles up the bone substitute material on a honeycomb like or porous basic structure of synthetic material, textile, metal, ceramics, carbon fibers tissue or bone cement.

12. A method according to one of the claims 1–11. characterized in that one compresses mechanically the bone substitute material in a dry or wet state, with or without heating.

**Revendications**

1. Procédé de fabrication de matériau de remplacement des os à partir d'un matériau contenant du collagène et un composant minéral, le matériau contenant le collagène étant réticulé, caractérisé en ce qu'une solution ou dispersion aqueuse de collagène nettoyé est utilisée, qui est mélangée avec le composant minéral consistant en apatite et/ou hydroxylapatite et/ou céramique de phosphate de calcium et une quantité d'un agent de réticulation produisant une réticulation partielle du collagène, la quantité de l'agent de réticulation utilisé étant dimensionnée de manière à être équivalente à la quantité de moins de 1% en poids de formaldéhyde par rapport au poids sec du collagène, de sorte que le matériau, après implantation dans le corps, ne donne pas lieu à des effets secondaires indésirables et qu'il conserve sa résorbabilité et son pouvoir absorbant vis-à-vis des liquides du corps; et en ce que le mélange est lyophilisé.

2. Procédé selon la revendication 1, caractérisé en ce que de l'apatite, plus particulièrement de l'hydroxylapatite frittée, resp. du phosphate de calcium avec un diamètre de 20–1000 µm, de préférence 50–300 µm sont utilisés comme composant minéral.

3. Procédé selon la revendication 1 ou 2, caractérisé en ce que le rapport en poids du collagène au composant minéral se monte de 1:2 à 1:10, de préférence de 1:3 à 1:5.

4. Procédé selon l'une des revendications 1 à 3, caractérisé en ce que l'on mélange une solution ou dispersion aqueuse du collagène nettoyé avec les grains du composant minéral, que l'on lyophilise le mélange et que l'on traite le lyophilisat obtenu avec un agent de réticulation gazeux.

5. Procédé selon la revendication 1, caractérisé en ce que l'on utilise du formaldéhyde ou un oxyde d'éthylène gazeux comme agent de réticulation gazeux.

6. Procédé selon la revendication 1, caractérisé en ce que l'on utilise comme agent de réticulation un aldéhyde aliphatique monofonctionnel ou polyfonctionnel saturé ou non saturé.

7. Procédé selon la revendication 6, caractérisé en ce que l'on utilise comme aldéhyde du formaldéhyde, glutaraldéhyde ou glyoxal.

8. Procédé selon la revendication 1, 6 ou 7, caractérisé en ce que pour la réticulation partielle du collagène, on utilise du formaldéhyde dans une quantité de 0.05–0,5% en poids par rapport au poids sec du collagène, ou la quantité équivalente de l'un des autres aldéhydes cités.

9. Procédé selon la revendication 1, caractérisé en ce que l'on utilise comme agent de réticulation du dichlortriazine ou trichlortriazine non substitué ou substitué.

10. Procédé selon l'une des revendications 1–9, caractérisé en ce que l'on ajoute un antibiotique au collagène, au composant minéral ou au matériau fabriqué avec ceux-ci.

11. Procédé selon l'une des revendications 1–10, caractérisé en ce que l'on empile le matériau de remplacement des os sur une structure de base en nid d'abeilles ou poreuse en matériau synthétique, textile, métal, céramique, tissu de fibres de carbone ou cément des os.

12. Procédé selon l'une des revendications 1 à 11, caractérisé en ce que l'on comprime mécaniquement le matériau de remplacement des os à sec, à l'état mouillé, avec ou sans chauffage.